# EUROPEAN PATENT APPLICATION

(11) **EP 4 613 262 A1**
(43) Date of publication of application: **10.09.2025**
(21) Application number: 23885680.1
(22) Date of filing: 27.10.2023
(51) Int. Cl.: A61K 8/81, A45D 31/00, A61K 8/46, A61K 8/55, A61Q 3/02

(54) **PHOTOCURABLE COMPOSITION FOR NAILS OR ARTIFICIAL NAILS**

(30) Priority: 04.11.2022 JP 2022177268
(71) Applicant: ThreeBond Co., Ltd., Tokyo 192-0398 (JP)
(72) Inventor: MORIKAWA, Yumi, Hachioji-shi, Tokyo 192-0398 (JP); HARADA, Natsumi, Hachioji-shi, Tokyo 192-0398 (JP)
(74) Representative: Crow, Martin
(86) International application number: PCT/JP2023/038945
(87) International publication number: WO 2024/095924

(57) **Abstract**

To provide a photocurable composition for a nail or an artificial nail, which is suppressed in heat generation and also satisfies both storage stability and photocurability and which allows gloss to be expressed on a surface of a cured product.

A photocurable composition for a nail or an artificial nail, the composition comprising components (A) to (D), and the composition comprising 30 to 70 parts by mass of the component (B) and 10 to 80 parts by mass of the component (C) based on 100 parts by mass of the component (A):
component (A): a urethane-modified (meth)acrylate oligomer having a weight average molecular weight of 3000 to 6000 and containing 3 to 5 (meth)acryloyl groups per molecule;
component (B): a (meth)acrylate containing one or more (meth)acryloyl groups per molecule (except for the component (A));
component (C): a polythiol compound; and
component (D): a photoinitiator.

## Description

### TECHNICAL FIELD

The present invention relates to a photocurable composition for a nail or an artificial nail.

### BACKGROUND ART

So-called gel nails have the property of providing cured products expressing gloss on surfaces thereof by light irradiation, and therefore are used for decoration or the like of nails or artificial nails. Procedures on nails or artificial nails are performed by directly or indirectly coating human natural nails with gel nails and irradiating such gel nails with light for curing. JP 2017-210475 describes the invention relating to a gel nail, and it is known that a polythiol compound is added to a compound having a (meth)acryloyl group, thereby allowing a gel nail to be enhanced in photocurability, accordingly hardly affected by oxygen inhibition at an interface in contact with oxygen, and enhanced in surface curability.

### SUMMARY OF INVENTION

However, it has been found that a gel nail, when includes a polythiol compound, has difficulties in allowing gloss to be expressed on a surface of a cured product and allowing durability of such gloss to be kept. It has also been found that an increase in photocurability due to inclusion of a polythiol compound in a gel nail tends to cause such a gel nail, which is left to still stand before procedures, to be increased in viscosity and decreased in storage stability. Although storage stability can be enhanced by addition of a polymerization inhibitor or the like, a problem is that gloss of a surface of a cured product is affected. Furthermore, heat generation may occur on a gel nail along with photocuring at the stage of light irradiation during nail procedures, and a subject may feel stress about heat.

Accordingly, an object of the present invention is to provide a photocurable composition for a nail or an artificial nail, which is suppressed in heat generation (curing heat) and also satisfies both storage stability and photocurability and which allows gloss to be expressed on a surface of a cured product.

The present inventor has made intensive studies in order to achieve the above object, and as a result, have completed the present invention relating to a photocurable composition.

The gist of the present invention is then described. A first embodiment of the present invention is a photocurable composition for a nail or an artificial nail, the composition comprising components (A) to (D), and the composition comprising 30 to 70 parts by mass of the component (B) and 10 to 80 parts by mass of the component (C) based on 100 parts by mass of the component (A):
component (A): a urethane-modified (meth)acrylate oligomer having a weight average molecular weight of 3000 to 6000 and containing 3 to 5 (meth)acryloyl groups per molecule;
component (B): a (meth)acrylate having one or more (meth)acryloyl groups per molecule (except for the component (A));
component (C): a polythiol compound; and
component (D): a photoinitiator.

A second embodiment of the present invention is the photocurable composition for a nail or an artificial nail according to the first embodiment, wherein the component (A) has a polyether backbone.

A third embodiment of the present invention is the photocurable composition for a nail or an artificial nail according to the first or second embodiment, wherein the component (B) is composed of only a monofunctional (meth)acrylate and/or a difunctional (meth)acrylate.

A fourth embodiment of the present invention is the photocurable composition for a nail or an artificial nail according to the third embodiment, wherein the monofunctional (meth)acrylate is a monofunctional (meth)acrylate having a hydroxyl group.

A fifth embodiment of the present invention is the photocurable composition for a nail or an artificial nail according to the third or fourth embodiment, wherein the difunctional (meth)acrylate is dimethylol tricyclodecane diacrylate.

A sixth embodiment of the present invention is the photocurable composition for a nail or an artificial nail according to any of the first to fifth embodiments, the composition further comprising a phosphorus-based compound as a storage stabilizer.

A seventh embodiment of the present invention is the photocurable composition for a nail or an artificial nail according to the sixth embodiment, wherein the phosphorus-based compound is a phosphonic acid compound.

An eighth embodiment of the present invention is the photocurable composition for a nail or an artificial nail according to any of the first to seventh embodiments, wherein the photocurable composition for a nail or an artificial nail is for topcoat layer formation.

The present invention can provide a photocurable composition which is suppressed in heat generation (curing heat) and also satisfies both storage stability and photocurability and which allows gloss to be expressed on a surface of a cured product.

### DESCRIPTION OF EMBODIMENTS

Hereinafter, embodiments of the present invention are described. The present invention is not limited only to the following embodiments. Herein, unless particularly noted, operations, and measurement of physical properties and the like are carried out in conditions of room temperature (20°C or more and 25°C or less) and a relative humidity of 40% RH or more and 50% RH or less. In addition, the "A and/or B" includes each of A and B, and any combination of one or more thereof, and specifically means at least one of A and B, and means A, B, and a combination of A and B. Herein, "X to Y" is used to include numerical values (X and Y) before and after "to" as a lower limit value and an upper limit value, and means "X or more and Y or less". In a case where the "X to Y" is plurally described, for example, in a case where "X1 to Y1, or X2 to Y2" is described, all of a disclosure where each numerical value is defined as the upper limit, a disclosure where each numerical value is defined as the lower limit, and a disclosure of any combination of such upper limit and lower limit are made (namely, serve as a legitimate basis for the amendment). Specifically, all of an amendment with X1 or more, an amendment with Y2 or less, an amendment with X1 or less, an amendment with Y2 or more, an amendment with X1 to X2, an amendment with X1 to Y2, and the like must be regarded as being legitimate.

One embodiment of the present invention provides a photocurable composition for a nail or an artificial nail, the composition comprising components (A) to (D), and the composition comprising 30 to 70 parts by mass of the component (B) and 10 to 80 parts by mass of the component (C) based on 100 parts by mass of the component (A):
component (A): a urethane-modified (meth)acrylate oligomer having a weight average molecular weight of 3000 to 6000 and containing 3 to 5 (meth)acryloyl groups per molecule;
component (B): a (meth)acrylate having one or more (meth)acryloyl groups per molecule (except for the component (A));
component (C): a polythiol compound; and
component (D): a photoinitiator.

Thus, there can be provided a photocurable composition comprising a compound having a (meth)acryloyl group and a polythiol compound, in which the composition is suppressed in heat generation during photocuring and also satisfies both storage stability and photocurability, and allows gloss to be expressed on a surface of a cured product. In other words, the photocurable composition of the present invention is suppressed in heat generation during photocuring, is excellent in storage stability, high in photocurability, and allows high gloss to be expressed on a surface of a cured product. In the present invention, the "having gloss" means a glossiness of more than 80% in measurement described below, whereas the "having luster" means a state where, when a surface is visually confirmed in many directions, the surface can be confirmed to be flat and smooth even in any direction and can be confirmed have gloss even in any direction.

The detail of the present invention is then described. The component (A) usable in the present invention is a urethane-modified (meth)acrylate oligomer having a weight average molecular weight of 3000 to 6000 and containing 3 to 5 (meth)acryloyl groups per molecule. Hereinafter, "acryloyl" and "methacryloyl" are collectively called "(meth)acryloyl". Herein, the weight average molecular weight refers to a weight average molecular weight in terms of polystyrene, as measured by gel permeation chromatography.

The urethane-modified (meth)acrylate oligomer is a (meth)acrylate oligomer having a urethane bond, and any oligomer having one or more urethane bonds and one or more (meth)acryloyl groups can be used. A urethane (meth)acrylate oligomer is added, to result in the effects of an enhancement in adhesiveness to a nail or an artificial nail and enhancements in curability and strength of a photocurable composition (coating film). The "oligomer" refers to a polymer (a polymer having a molecular weight of more than 1000) in which two to several tens of monomer units (including a monomer unit other than a (meth)acrylate monomer) are repeated. The urethane-modified (meth)acrylate oligomer may be synthesized by a known method, and there is known, for example, a synthesis in which a urethane bond is formed from a polyol and a polyisocyanate, and a compound having a hydroxyl group and a (meth)acryloyl group in its molecule or a (meth)acryloyl acid is added to the remaining isocyanate group. The component (A) in the present invention is a urethane-modified acrylate oligomer having a weight average molecular weight of 3000 to 6000 and containing 3 to 5 acryloyl groups per molecule, and thus a cured product resulting from curing of the photocurable composition can be enhanced in durability of gloss and photocurability is improved.

Examples of the polyol include a polyol compound having 2 or more hydroxyl groups in its molecule. Specific examples of the polyol include polyether polyol, polyester polyol, caprolactone diol, bisphenol polyol, polyisoprene polyol, hydrogenated polyisoprene polyol, polybutadiene polyol, hydrogenated polybutadiene polyol, castor oil polyol, and polycarbonate diol. In particular, the polyol is preferably polyether diol in order to introduce a polyether backbone into the component (A) from the viewpoint of expression of glossiness. These may be used singly or in combinations of a plurality thereof. In one embodiment, the polyol can have 3 or more, 4 or more, or 5 or more hydroxyl groups in its molecule, and can have 10 or less, 8 or less, 6 or less, 4 or less, or 3 or less hydroxyl groups in its molecule.

Examples of the polyisocyanate include a compound having 2 or more isocyanate groups in its molecule, and specific examples include an aromatic polyisocyanate, an alicyclic polyisocyanate, and an aliphatic polyisocyanate, but not limited thereto. Examples of the aromatic polyisocyanate include 2,4-tolylene diisocyanate, 2,6-tolylene diisocyanate, 1,3-xylylene diisocyanate, 1,4-xylylene diisocyanate, tetramethylxylylene diisocyanate, diphenylmethane diisocyanate, naphthalene-1,5- diisocyanate, and triphenylmethane triisocyanate. Examples of the alicyclic polyisocyanate include isophorone diisocyanate, bis(4-isocyanatocyclohexyl)methane, 1,3-bis(isocyanatomethyl)cyclohexane, 1,4-bis(isocyanatomethyl)cyclohexane, norbornane diisocyanate, and bicycloheptane triisocyanate. Examples of the aliphatic polyisocyanate include hexamethylene diisocyanate, 1,3,6-hexamethylene triisocyanate, and 1,6,11-undeca triisocyanate. In particular, diisocyanate such as isophorone diisocyanate or hexamethylene diisocyanate is preferred. Herein, the "aromatic polyisocyanate" means a polyisocyanate having an aromatic ring in its structure. Herein, the "alicyclic polyisocyanate" means a polyisocyanate having an alicyclic structure in its structure. Herein, the "aliphatic polyisocyanate" means a polyisocyanate in which a structure other than an isocyanate structure is constituted by a straight or branched aliphatic group.

Examples of the compound having a hydroxyl group and a (meth)acryloyl group include mono(meth)acrylates of dihydric alcohols such as ethylene glycol, propylene glycol, 1,3-propanediol, 1,3-butanediol, 1,4-butanediol, and polyethylene glycol, and mono(meth)acrylates or di(meth)acrylates of trihydric alcohols such as trimethylolethane, trimethylolpropane, and glycerin. These may be used singly or in combinations of a plurality thereof.

The component (A) used here is preferably a urethane (meth)acrylate oligomer of a polyether backbone, more preferably a difunctional urethane (meth)acrylate oligomer of a polyether backbone. Herein, other urethane (meth)acrylate oligomer, for example, a urethane (meth)acrylate oligomer of a polyester backbone, a urethane (meth)acrylate oligomer of a polycaprolactone backbone, a urethane (meth)acrylate oligomer of a polycarbonate backbone, or the like can also be used in combination.

The component (A) contains 3 to 5 (meth)acryloyl groups per molecule and has a weight average molecular weight of 3000 to 6000. Thus, rapid curing can be made without heat generation during photocuring. Furthermore, tackiness does not remain and luster is expressed on a surface of a cured product. In one embodiment, the component (A) contains 3 to 4 (meth)acryloyl groups per molecule. In one embodiment, the component (A) contains 3 to 5, or 3 to 4 methacryloyl groups per molecule. In one embodiment, the weight average molecular weight of the component (A) is 4000 to 5500, or 4900 to 5500. In one embodiment, the component (A) has a polyether backbone in its structure. The component (A) may be used singly or in combinations of two or more kinds thereof. In a case where two or more are used in combination, the total amount thereof is indicated as the content of the component (A).

In one embodiment, 3 to 5 (meth)acryloyl groups contained in the component (A) are located on each of both ends with a repeated structure being interposed. In one embodiment, the component (A) contains 3 or 4 (meth)acryloyl groups. In one embodiment, the component (A) is a compound represented by the following chemical formula 1: 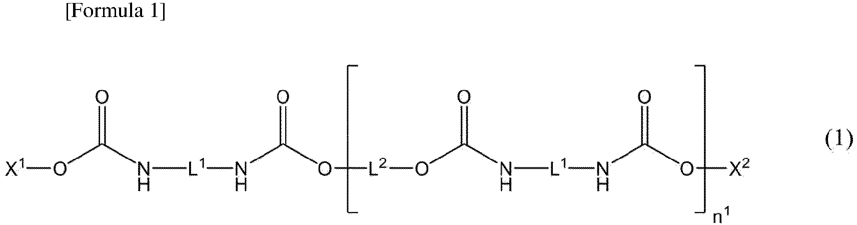 wherein,
L¹ is an alkylene group, a cycloalkylene group, an arylene group, or a group as a combination thereof,
L² is an alkylene group, an arylene group, a polyoxyalkylene group (*-[A¹-O]ₙ₂-A¹-*), a poly(oxycarbonylalkylene) group, a poly(oxycarbonyloxyalkylene) group, or a combination thereof,
   wherein A¹ is an ethylene group, a propylene group, a trimethylene group, or a tetramethylene group, and n2 is an integer of 0 to 100,
X¹ and X² are each independently a structure represented by the following: 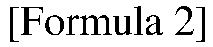 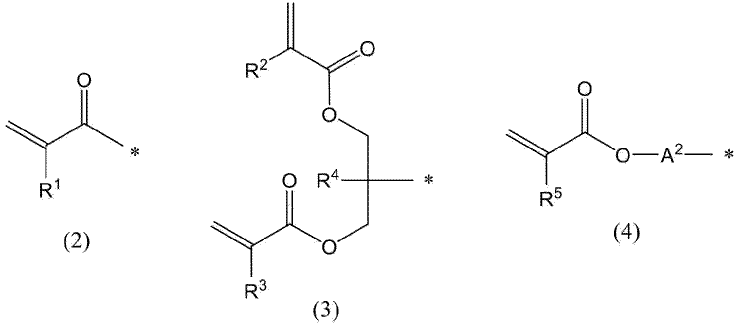
wherein,
   R¹ to R³ and R⁵ are each independently a hydrogen atom or a methyl group,
   R⁴ is a hydrogen atom, a methyl group, or an ethyl group,
   A² is an ethylene group, a propylene group, a trimethylene group, or a tetramethylene group, and
   in a case where any one of X¹ and X² is a structure represented by any one of (2) and (4), the other is a structure represented by (3), and
   n¹ is an integer of 1 to 100.

In one embodiment, L² in the chemical formula (1) contains a polyoxyalkylene group. In a case where the component (A) contains a molecule of such a structure, a cured product obtained by photocuring the photocurable composition can have gloss on a surface. In one embodiment, L² in the chemical formula (1) contains only a polyoxyalkylene group.

Herein, * represents a binding point with another atom or atom group. Herein, the "alkylene group" refers to a substituted or unsubstituted alkylene group having 1 to 20 carbon atoms, 1 to 15 carbon atoms, 1 to 10 carbon atoms, 2 to 8 carbon atoms, or 2 to 4 carbon atoms. Examples of the alkylene group include a methylene group, an ethylene group, a propylene group, a trimethylene group, a tetramethylene group, a pentamethylene group, and a hexamethylene group, but not limited thereto.

Herein, the "oxyalkylene group" is a group represented by formula (5):
[Formula 3]

*-O-A³-* Formula (5)

wherein A³ is an alkylene group. Examples of the oxyalkylene group include an oxyethylene group, an oxypropylene group, an oxytrimethylene group, and an oxytetramethylene group, but not limited thereto.

Herein, the "arylene group" refers to a substituted or unsubstituted arylene group having 6 to 30 carbon atoms, 6 to 20 carbon atoms, 6 to 15 carbon atoms, or 6 to 10 carbon atoms. Examples of the arylene group include a phenylene group, a toluidine-diyl group, a xylene-diyl group, and naphthylene group, but not limited thereto.

Herein, the "substituted" refers to a hydrogen atom being substituted with any other atom or a substituent. Examples of such an atom or substituent include, unless especially defined, a halogen atom; an alkyl group having 1 to 20 carbon atoms, 1 to 10 carbon atoms, or 1 to 6 carbon atoms; an alkoxy group having 1 to 20 carbon atoms, 1 to 10 carbon atoms, or 1 to 6 carbon atoms; an aryl group having 6 to 30 carbon atoms, 6 to 20 carbon atoms, 6 to 15 carbon atoms, or 6 to 10 carbon atoms; or any combination thereof, but not limited thereto.

Specific examples include Art Resin KY-11 manufactured by Negami Chemical Industrial Co., Ltd, but not limited thereto.

In one embodiment, the amount of the component (A) contained in the photocurable composition for a nail or an artificial nail comprises is 30% by mass or more, 40% by mass or more, or 45% by mass or more based on the total mass of the photocurable composition for a nail or an artificial nail. In one embodiment, the amount of the component (A) contained is 70% by mass or less, 60% by mass or less, 55% by mass or less, or 51% by mass or less based on the total mass of the photocurable composition for a nail. For example, the amount of the component (A) contained is preferably 30% by mass or more and 65% by mass or less, more preferably 35% by mass or more and 60% by mass or less, further preferably 40% by mass or more and 56% by mass or less based on the total mass of the photocurable composition for a nail or an artificial nail.

The component (B) usable in the present invention is a (meth)acrylate containing one or more (meth)acryloyl groups per molecule, provided that the component (A) is excluded. A compound having a (meth)acryloyl group can be used as the component (B). Specifically, the compound having a (meth)acryloyl group refers to a (meth)acrylate monomer or a (meth)acrylate oligomer. In one embodiment, the monomer has a molecular weight of 1000 or less. Herein, the "oligomer" refers to a compound having a molecular weight or weight average molecular weight of more than 1000, among compounds each having a structure in which a plurality of repeating units is bound, and such a compound itself, even if further formed into a multimer by polymerization or the like, is not referred to as "monomer", but referred to as "oligomer", unless otherwise described. The molecular weight of the monomer used as the component (B) can be 1000 or less, 800 or less, 500 or less, or 400 or less. In one embodiment, the weight average molecular weight (or molecular weight) of the oligomer used as the component (B) can be 2000 or more, 3000 or more, 4000 or more, 5000 or more, 6000 or more, 7000 or more, 10000 or more, 20000 or more, or 30000 or more, and can be 500000 or less, 300000 or less, 200000 or less, or 100000 or less. The component (B) is preferably in the form of a liquid under an atmosphere at 25°C, and can be suitably used as long as it has favorable compatibility with the components (A) and (C) in the present invention.

Specific examples of the (meth)acrylate oligomer used as the component (B) include a urethane-modified (meth)acrylate oligomer other than the component (A), a (meth)acrylate oligomer having an ester bond in its molecule, a (meth)acrylate oligomer having an ether bond in its molecule, and an epoxy-modified (meth)acrylate oligomer, and examples of a main backbone thereof include bisphenol A, novolac phenol, polybutadiene, polyester, and polyether, but not limited thereto. In one embodiment, the main backbone of the (meth)acrylate oligomer used as the component (B) contains a structure such as bisphenol A, novolac phenol, polybutadiene, polyester, or polyether, but not limited thereto. The component (A) usable in the present invention also encompasses a compound having one or more epoxy groups and one or more (meth)acryloyl groups in one molecule.

In one embodiment, the (meth)acrylate oligomer used as the component (B) contains 1 or 2 acryloyl groups in one molecule. In one embodiment, the (meth)acrylate oligomer used as the component (B) can contain 2 or more, 3 or more, 6 or more, or 8 or more, and 30 or less, 20 or less, or 15 or less acryloyl groups in one molecule.

The urethane-modified (meth)acrylate oligomer used as the component (B), other than the component (A), is a urethane-modified (meth)acrylate oligomer having a weight average molecular weight of less than 3000 or more than 6000, or containing less than 3 or more than 5 (meth)acryloyl groups per molecule.

A known method for synthesizing the (meth)acrylate oligomer having an ester bond used as the component (B) is a synthesis method including forming an ester bond by a polyol and a polyvalent carboxylic acid and adding acrylic acid to an unreacted hydroxyl group, but not limited to this synthesis method. Specific examples include Aronix (registered trademark, the same applies to the present specification) M-6100, M-6200, M-6250, M-6500, M-7100, M-7300K, M-8030, M-8060, M-8100, M-8530, M-8560, and M-9050 manufactured by Toagosei Co., Ltd., and UV-3500BA, UV-3520TL, UV-3200B, and UV-3000B manufactured by Nippon Synthetic Chemical Industry Co., Ltd., but not limited thereto.

A known method for synthesizing the (meth)acrylate oligomer having an ether bond, used as the component (B), is a synthesis method including adding acrylic acid to a hydroxyl group of a polyether polyol or a hydroxyl group of an aromatic polyether polyol such as bisphenol, but not limited to this synthesis method. The oligomer may have a urethane bond, together with an ether bond, in its molecule, or may be a urethane-modified (meth)acrylate oligomer having an ether bond. Specific examples include UV-6640B, UV-6100B, and UV-3700B manufactured by Nippon Synthetic Chemical Industry Co., Ltd., Light (Meth)acrylates 3EG-A, 4EG-A, 9EG-A, 14EG-A, PTMGA-250, BP-4EAL, BP-4PA, and BP-10EA manufactured by Kyoeisha Chemical Co., Ltd., and EBECRYL3700 manufactured by Daicel-Allnex Ltd., but not limited thereto.

The epoxy-modified (meth)acrylate oligomer used as the component (B) can be synthesized by applying (meth)acrylic acid or the like to a glycidyl group of a polyfunctional glycidyl ether compound, for ring-opening polymerization, but not limited thereto. Various types of backbones such as a bisphenol A type, a bisphenol F type, and a novolac phenol type can be used in a main chain of such a polyfunctional glycidyl ether. Specific examples of the above epoxy-modified acrylic oligomer include epoxy esters 3000A and 3002A manufactured by Kyoeisha Chemical Co., Ltd., and EBECRYL3700 manufactured by Daicel-Allnex Ltd., but not limited thereto.

The (meth)acrylate monomer used as the component (B) can include monofunctional, difunctional and trifunctional (meth)acrylate monomers. The component (B) used here can be a single monomer or a combination of a plurality of monomers. Herein, the term "functional group" refers to a group containing a (meth)acryloyl group, unless otherwise described, and, for example, the terms "monofunctional", "difunctional", and "trifunctional" can be respectively terms used for modifying compounds having 1, 2, and 3 (meth)acryloyl groups in their molecules. In one embodiment, the group having a (meth)acryloyl group can be a(meth)acryloyloxy group.

Specific examples of the monofunctional (meth)acrylate monomer used as the component (B) include lauryl (meth)acrylate, stearyl (meth)acrylate, ethyl carbitol (meth)acrylate, tetrahydrofurfuryl (meth)acrylate, caprolactone-modified tetrahydrofurfuryl (meth)acrylate, cyclohexyl (meth)acrylate, dicyclopentanyl (meth)acrylate, isobornyl (meth)acrylate, benzyl (meth)acrylate, phenyl (meth)acrylate, phenoxyethyl (meth)acrylate, phenoxydiethylene glycol (meth)acrylate, phenoxytetraethylene glycol (meth)acrylate, nonylphenoxyethyl (meth)acrylate, nonylphenoxytetraethylene glycol (meth)acrylate, methoxydiethylene glycol (meth)acrylate, ethoxydiethylene glycol (meth)acrylate, butoxyethyl (meth)acrylate, butoxytriethylene glycol (meth)acrylate, 2-ethylhexylpolyethylene glycol (meth)acrylate, 4-hydroxybutyl (meth)acrylate, nonylphenylpolypropylene glycol (meth)acrylate, methoxydipropylene glycol (meth)acrylate, 2-hydroxyethyl (meth)acrylate, 2-hydroxypropyl (meth)acrylate (or also referred to as 2-hydroxypropyl methacrylate), glycerol (meth)acrylate, polyethylene glycol (meth)acrylate, polypropylene glycol (meth)acrylate, epichlorohydrin-modified butyl (meth)acrylate, epichlorohydrin-modified phenoxy (meth)acrylate, N,N-dimethylaminoethyl (meth)acrylate, and N,N-diethylaminoethyl (meth)acrylate, but not limited thereto. The compound used as the component (B) preferably includes a monofunctional monomer having a hydroxyl group in one molecule. Specific examples include 2-hydroxyethyl (meth)acrylate and 2-hydroxypropyl (meth)acrylate, but not limited thereto.

Examples of the monofunctional (meth)acrylate monomer used as the component (B) also include a (meth)acrylate monomer having an acidic group. In one embodiment, the (meth)acrylate monomer having an acidic group particularly refers to a carboxylic acid, phosphoric acid or the like having a (meth)acryloyl group in its molecule. Examples of the carboxylic acid having a (meth)acryloyl group in its molecule include (meth)acryloyl acid, 3-(meth)acryloyloxypropylsuccinic acid, 4-(meth)acryloyloxybutylsuccinic acid, 2-(meth)acryloyloxyethylmaleic acid, 3-(meth)acryloyloxypropylmaleic acid, 4-(meth)acryloyloxybutylmaleic acid, 2-(meth)acryloyloxyethylhexahydrophthalic acid, 3-(meth)acryloyloxypropylhexahydrophthalic acid, 4-(meth)acryloyloxybutylhexahydrophthalic acid, 2-(meth)acryloyloxyethylphthalic acid, 3-(meth)acryloyloxypropylphthalic acid, and 4-(meth)acryloyloxybutylphthalic acid, and examples of the phosphoric acid having a (meth)acryloyl group in its molecule include 2-ethylhexyl acid phosphate, 2-hydroxyethyl methacrylate acid phosphate, and dibutyl phosphate, but not limited thereto. The (meth)acrylate monomer having an acidic group is preferably contained in order to enhance durability.

Specific examples of the difunctional (meth)acrylate monomer used as the component (B) include 1,3-butylene glycol di(meth)acrylate, 1,4-butylene glycol di(meth)acrylate, tricyclodecane dimethanol di(meth)acrylate, neopentyl glycol di(meth)acrylate, 1,6-hexane glycol di(meth)acrylate, ethylene glycol diacrylate, tetraethylene glycol di(meth)acrylate, polyethylene glycol di(meth)acrylate, propylene glycol di(meth)acrylate, tripropylene glycol di(meth)acrylate, dimethylol tricyclodecane di(meth)acrylate, ethylene oxide-modified neopentyl glycol di(meth)acrylate, propylene oxide-modified neopentyl glycol di(meth)acrylate, bisphenol A di(meth)acrylate, ethylene oxide-modified bisphenol A di(meth)acrylate, epichlorohydrin-modified bisphenol A di(meth)acrylate, ethylene oxide-modified bisphenol S di(meth)acrylate, neopentyl glycol-modified trimethylolpropane di(meth)acrylate, dicyclopentenyl di(meth)acrylate, ethylene oxide-modified dicyclopentenyl di(meth)acrylate, and diacryloyl isocyanurate, but not limited thereto. The difunctional (meth)acrylate monomer used as the component (B) includes one having a ring structure and one having no ring structure. In one embodiment, the difunctional (meth)acrylate monomer used as the component (B) has a ring structure. Examples of the ring structure include an alicyclic structure, an aromatic hydrocarbon ring structure, and a heterocyclic structure. A difunctional (meth)acrylate monomer having an alicyclic structure is preferably contained in consideration of curability. Examples of the alicyclic structure can include a monocyclic structure or a polycyclic structure. The polycyclic structure encompasses a ring-fused structure, and one having a ring-fused structure can be suitably used as the difunctional (meth)acrylate monomer used as the component (B). It is preferable to use dimethylol tricyclodecane di(meth)acrylate as the difunctional (meth)acrylate monomer having an alicyclic structure. The alicyclic structure is here a cycloaliphatic hydrocarbon structure, and specific examples include cycloalkyl such as cyclopropyl, cyclobutyl, cyclopentyl, cyclohexyl, butylhexyl, methylcyclohexyl, dimethylcyclohexyl, cycloheptyl, methylcycloheptyl, cyclooctyl, cyclononyl and cyclodecyl, hydronaphthyl, 1-adamantyl, 2-adamantyl, norbornyl, methylnorbornyl, isobornyl, dicyclopentenyl, dicyclopentanyl, dicyclopentenyloxyethyl, and tricyclodecane.

Specific examples of the trifunctional (meth)acrylate monomer include trimethylolpropane tri(meth)acrylate, trimethylolpropane tri(meth)acrylate, pentaerythritol tri(meth)acrylate, EO-modified trimethylolpropane tri(meth)acrylate, PO-modified trimethylolpropane tri(meth)acrylate, ECH-modified trimethylolpropane tri(meth)acrylate, ECH-modified glycerol tri(meth)acrylate, and tris(acryloyloxyethyl)isocyanurate, but not limited thereto.

Preferably, the monofunctional (meth)acrylate monomer includes a monofunctional (meth)acrylate having a hydroxyl group and the difunctional (meth)acrylate monomer includes a dimethylol tricyclodecane diacrylate from the viewpoint of surface curability and expression of gloss.

The component (B) preferably includes a monofunctional (meth)acrylate and/or a difunctional (meth)acrylate, is further preferably composed of only a monofunctional (meth)acrylate and/or a difunctional (meth)acrylate, and is most preferably composed of only a monofunctional (meth)acrylate and a difunctional (meth)acrylate. The component (B) preferably includes no tri- or higher functional (meth)acrylate from the viewpoint of suppression of heat generation (curing heat) during procedures.

The component (B) may be used singly or in combinations of two or more kinds thereof. In one embodiment, the component (B) includes a compound containing 1 or 2 (meth)acryloyl groups in one molecule. The component (B) may be composed of only a compound containing 1 and/or 2 (meth)acryloyl groups in one molecule. The component (B) may be composed of only a monomer or may be a mixture of a monomer and an oligomer. The monomer used as the component (B) may be used singly or in combinations of two or more kinds thereof, and the oligomer used as the component (B) may be used singly or in combinations of two or more kinds thereof. In a case where two or more kinds are used in combination, the total amount thereof is indicated as the content of the component (B).

In a preferred embodiment, the component (B) includes a (meth)acrylate monomer having 1 to 3 (meth)acryloyl groups per molecule. The (meth)acrylate monomer is preferably a (meth)acrylate monomer having 1 or 2 (meth)acryloyl groups per molecule. The (meth)acrylate monomer preferably includes one or more selected from the group consisting of a (meth)acrylate monomer having a hydroxyl group; a (meth)acrylate monomer having an acidic group; and a (meth)acrylate monomer having an alicyclic structure. The (meth)acrylate monomer more preferably includes one or more selected from the group consisting of a (meth)acrylate monomer having a hydroxyl group; and a (meth)acrylate monomer having an alicyclic structure. In other words, the component (B) preferably includes one or more selected from the group consisting of a (meth)acrylate monomer having a hydroxyl group and 1 or 2 (meth)acryloyl groups; and a (meth)acrylate monomer having an alicyclic structure and having 1 or 2 (meth)acryloyl groups.

In a case where the component (B) includes a (meth)acrylate monomer (b1) having a hydroxyl group and 1 or 2 (meth)acryloyl groups; and a (meth)acrylate monomer (b2) having an alicyclic structure and having 1 or 2 (meth)acryloyl groups, the mass ratio (b1:b2) of (b1) and (b2) is preferably 99:1 to 50:50, more preferably 95:5 to 65:45, further preferably 90:10 to 70:30.

In a preferred embodiment, the component (B) includes a urethane-modified (meth)acrylate oligomer other than the component (A). The urethane-modified (meth)acrylate oligomer is preferably a urethane-modified (meth)acrylate oligomer having a weight average molecular weight of less than 3000 or more than 6000 and having less than 3 or more than 5 (meth)acryloyl groups per molecule; more preferably a urethane-modified (meth)acrylate oligomer having a weight average molecular weight of more than 6000 and having 1 or 2 (meth)acryloyl groups per molecule.

In one embodiment, the component (B) includes one or more selected from the group consisting of a urethane-modified (meth)acrylate oligomer having a weight average molecular weight of more than 6000 and having 1 or 2 (meth)acryloyl groups per molecule; and a (meth)acrylate monomer having 1 or 2 (meth)acryloyl groups per molecule. In this case, the content of the urethane-modified (meth)acrylate oligomer is preferably 0.1 to 15 parts by weight, more preferably 0.5 to 10 parts by weight, further preferably 1 to 8 parts by weight, particularly preferably 1.5 to 5 parts by weight based on 100 parts by mass of the component (A). The content of the (meth)acrylate monomer is preferably 1 to 80 parts by weight, more preferably 5 to 70 parts by weight, further preferably 10 to 65 parts by weight, particularly preferably 20 to 60 parts by weight based on 100 parts by mass of the component (A). In a case where the component (B) includes the urethane-modified (meth)acrylate oligomer and the (meth)acrylate monomer, the mass ratio of urethane-modified (meth)acrylate oligomer : (meth)acrylate monomer is preferably 1:99 to 20:80, more preferably 2:98 to 15:85, further preferably 3:97 to 10:90.

In one embodiment, the component (B) includes one or more selected from the group consisting of a urethane-modified (meth)acrylate oligomer having a weight average molecular weight of more than 6000 and having 1 or 2 (meth)acryloyl groups per molecule; a (meth)acrylate monomer having a hydroxyl group and 1 or 2 (meth)acryloyl groups; and a (meth)acrylate monomer having an alicyclic structure and having 1 or 2 (meth)acryloyl groups.

The amount of the component (B) contained in the photocurable composition of the present invention is 30 to 70 parts by mass, most preferably 40 to 60 parts by mass based on 100 parts by mass of the component (A). When the amount of the component (B) contained is 30 parts by mass or more, the composition is lowered in viscosity and easily subjected to procedures, and when the amount of the component (B) contained is 70 parts by mass or less, curability is favorable. In a case where the component (B) is composed of a monofunctional (meth)acrylate and a difunctional (meth)acrylate, the mass ratio of monofunctional (meth)acrylate : difunctional (meth)acrylate is preferably 1:1 to 4:1, more preferably 2:1 to 3:1.

The amount of the (meth)acrylate oligomer used as the component (B) contained in the photocurable composition of the present invention can be 0 to 20% by mass or 5 to 10% by mass based on the total mass of the component (B). The amount of the (meth)acrylate oligomer used as the component (B) contained can be 0 to 10% by mass, 0.5 to 5% by mass, or 1 to 3% by mass based on the total mass of the photocurable composition.

The amount of the monofunctional (meth)acrylate monomer used as the component (B) contained in the photocurable composition of the present invention can be 50 to 100% by mass or 60 to 80% by mass based on the total mass of the component (B). The amount of the monofunctional (meth)acrylate monomer used as the component (B) contained can be 10 to 30% by mass or 15 to 25% by mass based on the total mass of the photocurable composition.

The amount of the difunctional (meth)acrylate monomer used as the component (B) contained can be 0 to 40% by mass or 10 to 30% by mass based on the total mass of the component (B). The amount of the difunctional (meth)acrylate monomer used as the component (B) contained can be 0 to 30% by mass or 5 to 20% by mass based on the total mass of the photocurable composition.

The component (C) usable in the present invention is a polythiol compound. The component (C) is not particularly limited as long as it has 2 or more thiol groups in one molecule, and may be used singly or in combinations of two or more kinds thereof. In one embodiment, the component (C) has 2 or 3, or 4 or more thiol groups in one molecule. The component (C) preferably has 3, or 4 or more thiol groups in one molecule from the viewpoint of an enhancement in surface curability. The component (C) preferably has 2 or 3 thiol groups in one molecule from the viewpoint of an enhancement in storage stability. Specific examples of the component (C) include an aliphatic polythiol compound, an aromatic polythiol compound, and a polythiol compound having a sulfide bond, but not limited thereto. In a case where the component (C) is an aliphatic polythiol group, the component has a primary thiol group, a secondary thiol group, and/or a tertiary thiol group, and preferably has a primary thiol group and/or a secondary thiol group, more preferably has a primary thiol group from the viewpoint of an enhancement in surface curability. In one embodiment, all of such thiol groups in the component (C) are each a primary thiol group and/or a secondary thiol group, and are preferably each a primary thiol group.

Examples of an aliphatic polythiol compound having two thiol groups include 1,2-ethanedithiol, 1,2-propanedithiol, 1,3-propanedithiol, 1,4-butanedithiol, 1,6-hexanedithiol, 1,7-heptanedithiol, 1,8-octanedithiol, 1,9-nonanedithiol, 1,10-decanedithiol, 1,12-dodecanedithiol, 2,2-dimethyl-1,3-propanedithiol, 3-methyl-1,5-pentanedithiol, 2-methyl-1,8-octanedithiol, 1,4-cyclohexanedithiol, 1,4-bis(mercaptomethyl)cyclohexane, 1,1-cyclohexanedithiol, 1,2-cyclohexanedithiol, bicyclo[2,2,1]hepta-exo-cis-2,3-dithiol, 1,1-bis(mercaptomethyl)cyclohexane, bis(2-mercaptoethyl)ether, ethylene glycol bis(2-mercaptoacetate), and ethylene glycol bis(3-mercaptopropionate), but not limited thereto.

Examples of an aliphatic polythiol compound having three thiol groups include 1,1,1-tris(mercaptomethyl)ethane, 2-ethyl-2-mercaptomethyl-1,3-propanedithiol, 1,2,3-propanetrithiol, trimethylolpropane tris(2-mercaptoacetate), trimethylolpropane tris(3-mercaptopropionate), and tris[(mercaptopropynyloxy)-ethyl]isocyanurate, but not limited thereto.

Examples of an aliphatic polythiol compound having four or more thiol groups include pentaerythritol tetrakis(2-mercaptoacetate), pentaerythritol tetrakis(3-mercaptopropionate), pentaerythritol tetrakis(3-mercaptobutyrate), and dipentaerythritol hexa-3-mercaptopropionate, but not limited thereto.

Examples of the aromatic polythiol compound include 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(2-mercaptoethyl)benzene, 1,3-bis(2-mercaptoethyl)benzene, 1,4-bis(2-mercaptoethyl)benzene, 1,2-bis(2-mercaptoethyleneoxy)benzene, 1,3-bis(2-mercaptoethyleneoxy)benzene, 1,4-bis(2-mercaptoethyleneoxy)benzene, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl)benzene, 1,3,5-tris(mercaptomethyl)benzene, 1,2,3-tris(2-mercaptoethyl)benzene, 1,2,4-tris(2-mercaptoethyl)benzene, 1,3,5-tris(2-mercaptoethyl)benzene, 1,2,3-tris(2-mercaptoethyleneoxy)benzene, 1,2,4-tris(2-mercaptoethyleneoxy)benzene, 1,3,5-tris(2-mercaptoethyleneoxy)benzene, 1,2,3,4-tetramercaptobenzene, 1,2,3,5-tetramercaptobenzene, 1,2,4,5-tetramercaptobenzene, 1,2,3,4-tetrakis(mercaptomethyl)benzene, 1,2,3,5-tetrakis(mercaptomethyl)benzene, 1,2,4,5-tetrakis(mercaptomethyl)benzene, 1,2,3,4-tetrakis(2-mercaptoethyl)benzene, 1,2,3,5-tetrakis(2-mercaptoethyl)benzene, 1,2,4,5-tetrakis(2-mercaptoethyl)benzene, 1,2,3,4-tetrakis(2-mercaptoethyleneoxy)benzene, 1,2,3,5-tetrakis(2-mercaptoethyleneoxy)benzene, 1,2,4,5-tetrakis(2-mercaptoethyleneoxy)benzene, 2,2'-mercaptobiphenyl, 4,4'-thiobis-benzenethiol, 4,4'-dimercaptobiphenyl, 4,4'-dimercaptobibenzyl, 2,5-toluenedithiol, 3,4-toluenedithiol, 1,4-naphthalenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-bis(2-mercaptoethylthio)benzene, 1,4-bis(2-mercaptoethylthio)benzene, 1,2-bis(2-mercaptoethylthiomethyl)benzene, 1,3-bis(2-mercaptoethylthiomethyl)benzene, 1,4-bis(2-mercaptoethylthiomethyl)benzene, 1,2,3-tris(2-mercaptoethylthio)benzene, 1,2,4-tris(2-mercaptoethylthio)benzene, 1,3,5-tris(2-mercaptoethylthio)benzene, 1,2,3,4-tetrakis(2-mercaptoethylthio)benzene, 1,2,3,5-tetrakis(2-mercaptoethylthio)benzene, and 1,2,4,5-tetrakis(2-mercaptoethylthio)benzene, but not limited thereto.

Examples of the polythiol compound having a sulfide bond include bis(2-mercaptoethyl)sulfide, bis(2-mercaptoethylthio)methane, 1,2-bis(2-mercaptoethylthio)ethane, 1,3-bis(2-mercaptoethylthio)propane, 1,2,3-tris(2-mercaptoethylthio)propane, tetrakis(2-mercaptoethylthiomethyl)methane, 1,2-bis(2-mercaptoethylthio)propanethiol, 2,5-dimercapto-1,4-dithiane, bis(2-mercaptoethyl)disulfide, 3,4-thiophenedithiol, 1,2-bis(2-mercaptoethyl)thio-3-mercaptopropane, and bis-(2-mercaptoethylthio-3-mercaptopropane)sulfide, but not limited thereto.

Examples of a commercially available product of the component (C) having a primary thiol group include PEMP, TMMP, TMMP-20P, DPMP, and TEMPIC manufactured by SC Organic Chemical Co., Ltd., but not limited thereto.

Specific examples of the component (C) having a secondary thiol group include pentaerythritol tetrakis(3-mercaptobutyrate), 1,4-bis(3-mercaptobutyryloxy)butane, 1,3,5-tris(3-mercaptobutyryloxyethyl)-1,3,5-triazin-2,4,6(1H,3H,5H)-trione, trimethylolpropane tris(3-mercaptobutyrate), trimethylolethane tris(3-mercaptobutyrate), trimethylolpropane tris(3-mercaptobutyrate), and trimethylolethane tris(3-mercaptobutyrate), but not limited thereto. Examples of a commercial product include PEMP manufactured by SC Organic Chemical Co., Ltd., and KarenzMT (registered trademark) series, PE1, BD1, and NR1 manufactured by Showa Denko K.K., but not limited thereto.

The amount of the component (C) contained is 10 to 80 parts by mass, preferably 10 to 70 parts by mass, more preferably 15 to 65 parts by mass, further preferably 20 to 60 parts by mass, particularly preferably 22 to 55 parts by mass, most preferably 25 to 50 parts by mass based on 100 parts by mass of the component (A). When the amount of the component (C) contained is 10 parts by mass or more, surface curability is enhanced, and when the amount is 80 parts by mass or less, storage stability is enhanced. When the content of the component (C) is less than 10 parts by mass, surface curability is not sufficient, and when the content is more than 80 parts by mass, curing heat is generated to sometimes cause stress to a user. The amount of the component (C) is preferably more than 8% by mass and less than 30% by mass based on the total mass of the composition. In a case where two or more kinds are used in combination, the total amount thereof is indicated as the content of the component (C).

The component (D) usable in the present invention is a photoinitiator (which is, namely, a photopolymerization initiator, and can be herein referred to as "photoinitiator"). The component (D) is not limited as long as it is a radical photopolymerization initiator which generates radical species by an energy ray such as visible light, ultraviolet light, X-ray, or electron beam. In one embodiment, the component (D) contains an ultraviolet light type photoinitiator and/or a visible light type photoinitiator. Here, the ultraviolet light type photoinitiator refers to a photopolymerization reaction initiator which absorbs ultraviolet light and thus generates radical species, and the visible light type photoinitiator is a photoinitiator which most strongly absorbs light in the visible light region.

Specific examples of the ultraviolet light type photoinitiator used as the component (D) include acetophenones such as diethoxyacetophenone, 2-hydroxy-2-methyl-1-phenylpropan-1-one, benzyl dimethyl ketal, 4-(2-hydroxyethoxy)phenyl-(2-hydroxy-2-propyl)ketone, 1-hydroxycyclohexyl phenyl ketone, 2-methyl-2-morpholino(4-thiomethylphenyl)propan-1-one, 2-benzyl-2-dimethylamino-1-(4-morpholinophenyl)butanone, and a 2-hydroxy-2-methyl-1-[4-(1-methylvinyl)phenyl]propanone oligomer; benzoins such as benzoin, benzoin methyl ether, benzoin ethyl ether, benzoin isopropyl ether, and benzoin isobutyl ether; benzophenones such as benzophenone, methyl o-benzoylbenzoate, 4-phenylbenzophenone, 4-benzoyl-4'-methyl-diphenyl sulfide, 3,3',4,4'-tetra(t-butylperoxycarbonyl)benzophenone, 2,4,6-trimethylbenzophenone, 4-benzoyl-N,N-dimethyl-N-[2-(1-oxo-2-propenyloxy)ethyl]benzenemethanaminium bromide, and (4-benzoylbenzyl)trimethylammonium chloride; and thioxanthones such as 2-isopropylthiothioxanthone, 4-isopropylthiothioxanthone, 2,4-diethylthiothioxanthone, 2,4-dichlorothiothioxanthone, 1-chloro-4-propoxythiothioxanthone, and 2-(3-dimethylamino-2-hydroxy)-3,4-dimethyl-9H-thiothioxanthone-9-one-mesochloride, but not limited thereto. The visible light type photoinitiator used as the component (D) is mainly an acyl phosphine oxide-based photopolymerization initiator containing a phosphorus atom, and specific examples include 2,4,6-trimethylbenzoyl-diphenyl-phosphine oxide and bis(2,4,6-trimethylbenzoyl)-phenylphosphine oxide, but not limited thereto. The component (D) may be used singly or in combinations of two or more kinds thereof. In a case where two or more kinds are used in combination, the ultraviolet light type photoinitiator and the visible light type photoinitiator may be used in combination as the component (D). In a case where two or more are used in combination, the total amount thereof is indicated as the content of the component (D).

The content of the component (D) is preferably 0.1 to 20 parts by mass, more preferably 1 to 18 parts by mass, further preferably 5 to 15 parts by mass based on 100 parts by mass of the component (A). When the content of the component (D) is 0.1 parts by mass or more, photocurability can be maintained. On the other hand, when the content of the component (D) is 20 parts by mass or less, storage stability can be maintained without thickening during storage. The amount of the visible light type photoinitiator contained is preferably 0 to 70% by mass based on the total mass of the component (D), and yellowing of a cured product is hardly caused. In one embodiment, the amount of the visible light type photoinitiator contained is preferably 10 to 60% by mass, more preferably 30 to 50% by mass based on the total mass of the component (D).

A proper amount of additive(s) such as a (meth)acrylamide monomer, a storage stabilizer, a coupling agent, an inorganic filling agent or an organic filling agent, a colorant such as a pigment or a dye, an antioxidant, a polymerization inhibitor, a defoaming agent, a leveling agent, and/or a rheology control agent may be compounded in the photocurable composition of the present invention, as long as features of the present invention are not impaired. Such addition provides a composition excellent in resin strength, workability, storage stability, and the like, or a cured product thereof.

A (meth)acrylamide monomer can be added to the photocurable composition of the present invention as long as features of the present invention are not impaired. Specific examples include dimethyl(meth)acrylamide, (meth)acryloylmorpholine, and diethyl(meth)acrylamide, but not limited thereto. Although no clear reason is found, the monomer preferably contains the (meth)acrylamide monomer from the viewpoint of an enhancement in durability. DMAA, ACMO, DEAA, and the like manufactured by KJ Chemicals Corporation are known as specific examples of the (meth)acrylamide monomer in the present invention, without limitation thereto.

A storage stabilizer can be added to the photocurable composition of the present invention, as long as features of the present invention are not impaired. The storage stabilizer is a storage stabilizer having a pKa (acid dissociation constant) of 1.0 to 4.0, and does not include the component (A) and the component (B). The storage stabilizer is more specifically an inorganic acid and/or an organic acid having a pKa of 1.0 to 4.0. The storage stabilizer is preferably an organic acid from the viewpoint of compatibility with other components. Although no clear reason is found, the storage stabilizer is added to the composition according to the present invention to allow both storage stability such as viscosity and surface curability to be enhanced. The pKa is one index quantitatively representing the strength of acid (ease of dissociation of hydrogen ion), and it can be said that a lower pKa means a stronger acid. The pKa is known to be measured by neutralization titration, absorption photometry, capillary electrophoresis, or the like, and neutralization titration is highest in accuracy among them. The storage stabilizer is particularly preferably a phosphorus compound serving as the storage stabilizer. Herein, those called phosphorus compound include phosphonic acid and phosphoric acid, provided that the component (D) is excluded. Most preferred is a phosphonic acid compound. The phosphonic acid compound is represented by R¹-P(=O)(OR²)₂, wherein R¹ is hydrogen or an organic group not bound with a phosphorus atom via an oxygen atom, and each R² independently represents hydrogen or an organic group. Examples of the organic group include an alkyl group having 1 to 20 carbon atoms and an aromatic group having 6 to 20 carbon atoms, and examples include a phenyl group and a hydrocarbon group, but not limited thereto. The storage stabilizer is most preferably phenylphosphonic acid. The storage stabilizer may be used singly or in combinations of a plurality thereof, and the composition preferably does not include any other storage stabilizer than the storage stabilizer. In general, phosphoric acid is represented by P(=O)(OH)₃ and differs from H-P(=O)(OH)₂ as phosphonic acid.

Specific examples of the storage stabilizer include phosphoric acid (pKa = 1.83), oxalic acid (pKa = 1.04), and a phosphonic acid compound, and examples of the phosphonic acid compound particularly include phenylphosphonic acid (pKa = 1.83), vinylphosphonic acid (pKa = 2.11), and methylphosphonic acid (pKa = 2.38), but not limited thereto. The storage stabilizer may be used singly or in combinations of a plurality thereof. It is preferable not to use any organic acid having a pKa of more than 4.0, other than the storage stabilizer, in order not to reduce storage stability in terms of viscosity or the like.

The amount of the storage stabilizer added is preferably 0.01 to 5.0 parts by mass, in particular, most preferably 0.01 to 2.0 parts by mass based on 100 parts by mass of the component (A). When the content of the storage stabilizer is 0.01 parts by mass or more, the change in viscosity is suppressed, and when the content is 5.0 parts by mass or less, surface curability is maintained. The content of the storage stabilizer is preferably 0.01 to 10.0% by mass based on the total mass of the composition. In a case where two or more are used in combination, the total amount thereof is indicated as the content of the storage stabilizer.

In the present invention, a coupling agent can be added as long as features of the present invention are not impaired. Examples of the coupling agent include a silane-based coupling agent having an epoxy group, a vinyl group, an acryloyl group or a methacryloyl group, and also a hydrolyzable silane group in combination, a polyorganosiloxane having a phenyl group and a hydrolyzable silyl group, and/or a polyorganosiloxane having an epoxy group and a hydrolyzable silyl group, but not limited thereto. Specific examples of the silane-based coupling agent include allyltrimethoxysilane, vinyltrimethoxysilane, 3-glycidoxypropyltrimethoxysilane, 3-methacryloxypropyltrimethoxysilane, 3-acryloxypropyltrimethoxysilane, and 3-chloropropyltrimethoxysilane, but not limited thereto.

A filling agent such as an inorganic filling agent and an organic filling agent can be added in the photocurable composition of the present invention, as long as features of the present invention are not impaired. Such a filling agent can be added to adjust not only viscous properties/thixotropy, but also curability and toughness. Examples of the inorganic filling agent include alumina, silica, and amorphous silica, but not limited thereto. On the other hand, examples of the organic filling agent include a styrene filler, a rubber filler, and a core-shell acrylic filler, but not limited thereto. Examples of a specific product of silica include FUSELEX E-1 manufactured by Tatsumori Ltd. and AO-802 manufactured by Admafine, and examples of a specific product of amorphous silica include Aerosil series, 200 (not treated), R972 (treated with dimethyldichlorosilane), R976 (treated with dimethyldichlorosilane), RY200 (treated with dimethylsilicone), RX200 (treated with hexamethyldisilazane), and R800 (treated with octylsilane) manufactured by Nippon Aerosil Co., Ltd., but not limited thereto.

The method for preparing the photocurable composition for a nail of the present invention is not particularly limited, and a conventionally known method can be appropriately adopted. For example, the component (A), the component (B), the component (C), the component (D), and a component optionally added are respectively weighed in predetermined amounts, added to a stirring oven sequentially in any order or simultaneously, and then mixed by use of a mixing unit such as a planetary mixer preferably with defoaming in vacuum. A preferred order of addition is set so that the component (D) is added at the end. The component (D) can be added at the end to prevent photopolymerization reaction from progressing at an undesired stage. Here, production conditions are not particularly limited, and production is preferably performed under light shielding conditions. The mixing temperature is preferably a temperature of 10 to 50°C, and the mixing time is preferably 0.1 to 5 hours.

When the photocurable composition for a nail or an artificial nail of the present invention is used in a so-called gel nail, procedures are performed as follows. Before such procedures, sanding of the surface of a human nail by a file or the like is performed and then any dust, oil, water, and the like are removed by a nail-specific solvent mainly containing ethanol. Preferably, in the case of coating with the photocurable composition of the present invention, a coating film having a thickness of 100 to 300 µm before curing is formed by a pencil or a brush. A primer may also be used in advance in the coating. A commercially available LED lamp for gel nails is used as the irradiation apparatus in curing. In one embodiment, the wavelength of light used for light irradiation can be 200 to 800 nm, 250 to 700 nm, 280 to 600 nm, 300 to 500 nm, or 350 to 400 nm. The irradiation time is 15 seconds to 120 seconds, and is preferably 20 to 70 seconds in consideration of the influence on a finger.

The viscosity at 25°C of the photocurable composition for a nail or an artificial nail of the present invention is preferably 10 Pa·s or less, more preferably 5 Pa·s or less in consideration of coatability during procedures.

The photocurable composition for a nail or an artificial nail of the present invention is suppressed in heat generation (curing heat) during photocuring, namely, light irradiation, and thus a subject receiving procedures does not feel stress about heat. In one embodiment, heat generation (curing heat) during light irradiation of the photocurable composition for a nail or an artificial nail of the present invention is evaluated by a sensitivity test. The sensitivity test can be performed, for example, according to three-level evaluation criteria of "no hotness felt", "slightly warm", and "hot", by a subject receiving procedures. In one embodiment, the temperature after rising by heat generation (curing heat) during light irradiation of the photocurable composition for a nail or an artificial nail of the present invention can be preferably 50°C or less, more preferably less than 45°C, further preferably 40°C or less, still further preferably 36°C or less. The above temperature can be measured with, for example, a contactless thermometer.

It is known that polymerization is suppressed by oxygen inhibition in a region where a compound having a (meth)acryloyl group is in touch with oxygen when polymerized with generation of radical species by an energy ray. An uncured component here remains on a cured product surface, thus such an uncured component is often wiped off by a waste cloth or the like after photocuring during procedures to provide gloss, and such an operation is more likely performed particularly with respect to a topcoat used for the outermost surface. However, the photocurable composition according to the present invention is unlikely affected by oxygen inhibition during photocuring and thus is suitable for a non wipe topcoat where no uncured component remains on the surface after curing.

The initial glossiness of a cured product obtained by photocuring the photocurable composition for a nail or an artificial nail according to the present invention is preferably 85% or more, more preferably 87% or more, further preferably 88% or more. The glossiness after a wear test of a cured product obtained by photocuring the photocurable composition for a nail or an artificial nail according to the present invention is preferably 83% or more, more preferably 85% or more, further preferably 88% or more. The change in glossiness before and after the wear test is here preferably 5.8% or less, more preferably 4.0% or less, further preferably 3.0% or less, still further preferably 2.0% or less. Herein, the wear test is performed by a method described in Examples. Herein, measurement of the glossiness can be achieved by a method described in Examples.

In the present invention, while the numerical value obtained by measurement of the glossiness with a high gloss checker manufactured by Horiba Ltd., serves as one for reference purposes of the degree of gloss of a cured product, the presence or absence of gloss by visual evaluation of the entire surface of such a cured product is regarded as being definitely evaluated as an actual degree of gloss. Accordingly, whether the result of visual evaluation of surface gloss is Good or Poor is preferentially considered as gloss evaluation. For example, when the gloss of a plurality of cured products evaluated as Good is compared, the glossiness calculated with the high gloss checker can serve as an index, to compare the degree of gloss. When the gloss of a plurality of cured products evaluated as Poor is again compared, the glossiness calculated with the high gloss checker can serve as an index, to compare the degree of gloss.

### Examples

Next, the present invention is further specifically described with reference to Examples, but the present invention is not limited only to these Examples.

### [Examples 1 to 6 and Comparative Examples 1 to 4]

The following components were provided for preparing a photocurable composition (hereinafter, the photocurable composition for a nail or an artificial nail being also simply referred to as "composition".).
Component (A): urethane-modified (meth)acrylate oligomer having a weight average molecular weight of 3000 to 6000 and containing 3 to 5 (meth)acryloyl groups per molecule
   - Polyether-based urethane acrylate oligomer having a weight average molecular weight of 5000 and the number of functional groups of 3 (Art Resin KY-11 manufactured by Negami Chemical Industrial Co., Ltd.)
Urethane-modified (meth)acrylate oligomer other than component (A)
   · Polyether urethane acrylate having a weight average molecular weight of 38000 and the number of functional groups of 2 (SHIKOH UV-3700B Mitsubishi Chemical Corporation)
   · Polyether urethane acrylate having a weight average molecular weight of 3500 and the number of functional groups of 2 (Art Resin UN-6303 manufactured by Negami Chemical Industrial Co., Ltd.)
   · Urethane acrylate oligomer having a weight average molecular weight of 4900 and the number of functional groups of 10 (Art Resin UN-904 manufactured by Negami Chemical Industrial Co., Ltd.)
Component (B): (meth)acrylate having one or more (meth)acryloyl groups per molecule (except for component (A))
   - 2-Hydroxypropyl methacrylate (HPMA manufactured by Nippon Shokubai Co., Ltd.)
   - Dimethylol tricyclodecane diacrylate (Light Acrylate DCP-A manufactured by Kyoeisha Chemical Co., Ltd.)
Component (C): polythiol compound
   - Trimethylol propane tris(3-mercaptopropionate) (TMMP-20P manufactured by SC Organic Chemical Co., Ltd.)
Component (D): photoinitiator
   - 1-Hydroxycyclohexyl phenyl ketone (non-visible light type photoinitiator) (IRGACURE 184, manufactured by BASF SE)
   - 2,4,6-Trimethylbenzoyl-diphenyl-phosphine oxide (visible light type photoinitiator) (LUCIRIN TPO manufactured by BASF SE)
Storage stabilizer
   - Phenylphosphonic acid (reagent)

Preparation was performed in Examples 1 to 6 and Comparative Examples 1 to 4. Each composition was obtained by weighing the component (A) (and other oligomer), the component (B), the component (C) and the storage stabilizer in a stirring oven and then stirring them for 30 minutes, thereafter stirring them for 30 minutes with defoaming in vacuum, and finally weighing the component (D) and adding it into the stirring oven and stirring them for 30 minutes. The detailed amounts of preparation are as shown in Table 1, and all numerical values are expressed by "part(s) by mass".

**[Table 1]**

| Component | Raw material name | Example 1 | Example 2 | Example 3 | Example 4 | Example 5 | Example 6 | Comparative Example 1 | Comparative Example 2 | Comparative Example 3 | Comparative Example 4 |
|---|---|---|---|---|---|---|---|---|---|---|---|
| Component (A) | KY-11 | 100.0 | 100.0 | 100.0 | 100.0 | 100.0 | 100.0 | 100.0 | 100.0 | | |
| Urethane-modified (meth)acrylate oligomer other than component (A) | UV-3700B | | 3.1 | 3.1 | 3.1 | 3.1 | 3.1 | 3.1 | 3.1 | 3.1 | 9.4 |
| | UN-6303 | | | | | | | | | 100.0 | |
| | UN-904 | | | | | | | | | | 100.0 |
| Component (B) | HPMA | 40.6 | 40.6 | 56.3 | 40.6 | 40.6 | 40.6 | 40.6 | 40.6 | 40.6 | 40.6 |
| | DCP-A | 15.6 | 15.6 | | 15.6 | 15.6 | 15.6 | 15.6 | 15.6 | 15.6 | 15.6 |
| Component (C) | TMMP-20P | 27.2 | 15.6 | 27.2 | 27.2 | 46.9 | 62.5 | | 7.8 | 27.2 | 27.2 |
| Component (D) | 184 | 5.5 | 5.5 | 5.5 | 5.5 | 5.5 | 5.5 | 5.5 | 5.5 | 5.5 | 5.5 |
| | TPO | 4.7 | 4.7 | 4.7 | 4.7 | 4.7 | 4.7 | 4.7 | 4.7 | 4.7 | 4.7 |
| Storage stabilizer | Phenylphosphonic acid | 0.2 | 0.2 | 0.2 | 0.1 | 0.2 | 0.2 | 0.2 | 0.2 | 0.2 | 0.2 |
| Total | | 193.8 | 185.3 | 197.0 | 196.8 | 216.6 | 232.2 | 169.7 | 177.5 | 196.9 | 203.2 |

In Examples 1 to 6 and Comparative Examples 1 to 4, confirmation of the storage stability, confirmation of the cured product surface state, confirmation of the surface curability, measurement of the glossiness, the wear test, and confirmation of heat generation during procedures were performed. The results are summarized in Table 2.

### [Confirmation of storage stability]

A glass container was filled with 100 g of the composition, lidded and sealed, and thereafter left to still stand in a hot air dying furnace at 60°C for 3 days. Thereafter, the state of the composition is visually confirmed according to the following evaluation criteria, and is determined as "storage stability".

### Evaluation criteria

Good: fluidity confirmed in container inclined
Poor: no fluidity confirmed in container inclined (gel formed).

### [Confirmation of cured product surface state]

An acrylic plate of 2.0 mm thickness × 25 mm width × 100 mm length was coated with the composition by a brush so that the thickness of the composition was 300 µm. The composition was cured by irradiation with an UV lamp for nails (rated voltage: 100 to 110 V, consumed power at 50 to 60 Hz: 36 W, wavelength: 350 to 400 nm) for 30 seconds. The resultant is subjected to reflection onto a LED stand, and visually confirmed according to the following evaluation criteria, and "surface gloss" is determined.

### Evaluation criteria

Good: luster on surface
Poor: no luster on surface.

### [Confirmation of surface curability]

An acrylic plate of 2.0 mm thickness × 25 mm width × 100 mm length was coated with the composition by a brush so that the thickness of the composition was 300 µm. The composition was cured by irradiation with an UV lamp for nails (rated voltage: 100 to 110 V, consumed power at 50 to 60 Hz: 36 W, wavelength: 350 to 400 nm) for 30 seconds. The state of a cured product surface is visually confirmed according to the following evaluation criteria, with the surface being in contact with a cotton swab, and is determined as "surface curability".

### Evaluation criteria

Good: no tackiness of components occurring on surface
Poor: tackiness of components occurring on surface.

### [Measurement of glossiness]

A test piece (size: 0.8 mm thickness × 70 mm width × 150 mm length) where one surface of SPCC-SD as a substrate was subjected to electrodeposition coating with clear amino alkyd paint was used, the surface subjected to electrodeposition coating was coated with the composition at a thickness of 100 µm, and the composition was cured by irradiation with an UV lamp for nails (rated voltage: 100 to 110 V, consumed power at 50 to 60 Hz: 36 W, wavelength: 350 to 400 nm) for 30 seconds, to produce a test piece. The "initial glossiness (%)" of the surface of the test piece was measured with a high gloss checker manufactured by Horiba Ltd., at an angle of incidence 60° and an angle of light reception of 60°. The initial glossiness is preferably 85% or more for generation of luster in the appearance.

### [Wear test]

The surface of the test piece subjected to measurement according to the above Measurement of glossiness was wiped 100 times by a waste cloth, and again subjected to the same measurement as the Measurement of glossiness, and "glossiness after wear (%)" was determined. The "percentage of change in glossiness (%)" is determined by "Percentage of change in glossiness (%)" = ("Glossiness after wear (%)" - "Initial glossiness (%)")/"Initial glossiness (%)" × 100. The glossiness after wear is preferably 85% or more from the viewpoint of durability. The percentage of change in glossiness is preferably -6.0 to 0.0%, most preferably -3.0 to 0.0%.

### [Evaluation of heat generation during procedures]

After sanding of a nail is carried out, any dust and oil content on the surface of the nail are removed by a nail dedicated solvent (mainly containing ethanol). The nail was coated with a basecoat agent so that the wet thickness was about 100 µm. The coating was performed by a brush. Thereafter, the composition was cured with irradiation by a LED lamp for nails (rated voltage: 100 to 110 V, consumed power at 50 to 60 Hz: 36 W, wavelength: 350 to 400 nm) for 30 seconds. The same method is performed to sequentially coat the surface of the basecoat with a colorcoat and a topcoat, and these are sequentially cured under the same conditions. The colorcoat used was Super Color EX (color: pastel peach) manufactured by PREGEL, and the topcoat used was each composition. "Heat generation during procedures" was evaluated with respect to fingernails (10 fingers) of the hand of one person, according to the following evaluation criteria. "Good" is preferred in consideration of stress on a subject. Herein, the "during procedures" refers to a period of light irradiation during curing of each composition for formation of a topcoat.

### Evaluation criteria

Good: no hotness felt
Fair: slightly warm
Poor: hot

**[Table 2]**

| Test item | Example 1 | Example 2 | Example 3 | Example 4 | Example 5 | Example 6 | Comparative Example 1 | Comparative Example 2 | Comparative Example 3 | Comparative Example 4 |
|---|---|---|---|---|---|---|---|---|---|---|
| Storage stability | Good | Good | Good | Good | Good | Good | Good | Good | Good | Poor |
| Surface gloss | Good | Good | Good | Good | Good | Good | Good | Good | Poor | Good |
| Surface curability | Good | Good | Good | Good | Good | Good | Poor | Poor | Good | Poor |
| Initial glossiness | 90 | 88 | 88 | 88 | 90 | 90 | 87 | 85 | 83 | 90 |
| Glossiness after wear | 88 | 87 | 83 | 87 | 90 | 88 | 87 | 80 | 80 | 86 |
| Percentage of change in glossiness | -2.2 | -1.1 | -5.7 | -1.1 | 0.0 | -2.2 | 0.0 | -5.9 | -3.6 | -4.4 |
| Heat generation after procedures | Good | Good | Good | Good | Good | Good | Good | Good | Fair | Poor |

It was found from comparison between Examples 1 to 6 and Comparative Examples 1 and 2 that surface curability was enhanced by 10 to 80 parts by mass of the component (C) contained based on 100 parts by mass of the component (A). Examples 1 to 6, in which the urethane-modified (meth)acrylate oligomer having a weight average molecular weight of 3000 to 6000 and containing 3 to 5 (meth)acryloyl groups per molecule was used as the component (A), exhibited suppressed heat generation during procedures as compared with Comparative Examples 3 and 4 in which any other urethane-modified (meth)acrylate oligomer was used. Furthermore, Examples 1 to 6 tended to exhibit a low percentage of change in glossiness after wear as compared with Comparative Example 2.

### Industrial Applicability

The present invention is a photocurable composition which, although comprises polythiol, satisfies both surface curability and storage stability. In particular, although the change in viscosity, if occurs in procedures in the nail field, has an influence on coatability, the photocurable composition according to the present invention can be stably subjected to procedures. The present invention can also allow for no heat generation during procedures and furthermore favorable surface curability, and therefore allow for use as a non wipe topcoat.

The present application is based on Japanese Patent Application No. 2022-177268 filed on November 4, 2022, the disclosure of which is incorporated by reference in its entirety.

## Claims

1. A photocurable composition for a nail or an artificial nail, the composition comprising components (A) to (D), and the composition comprising 30 to 70 parts by mass of the component (B) and 10 to 80 parts by mass of the component (C) based on 100 parts by mass of the component (A):
component (A): a urethane-modified (meth)acrylate oligomer having a weight average molecular weight of 3000 to 6000 and containing 3 to 5 (meth)acryloyl groups per molecule;
component (B): a (meth)acrylate having one or more (meth)acryloyl groups per molecule (except for the component (A));
component (C): a polythiol compound; and
component (D): a photoinitiator.

2. The photocurable composition for a nail or an artificial nail according to claim 1, wherein the component (A) has a polyether backbone.

3. The photocurable composition for a nail or an artificial nail according to claim 1, wherein the component (B) is composed of only a monofunctional (meth)acrylate and/or a difunctional (meth)acrylate.

4. The photocurable composition for a nail or an artificial nail according to claim 3, wherein the monofunctional (meth)acrylate is a monofunctional (meth)acrylate having a hydroxyl group.

5. The photocurable composition for a nail or an artificial nail according to claim 3, wherein the difunctional (meth)acrylate is dimethylol tricyclodecane diacrylate.

6. The photocurable composition for a nail or an artificial nail according to claim 1, further comprising a phosphorus-based compound as a storage stabilizer.

7. The photocurable composition for a nail or an artificial nail according to claim 6, wherein the phosphorus-based compound is a phosphonic acid compound.

8. The photocurable composition for a nail or an artificial nail according to claim 1, wherein the photocurable composition for a nail or an artificial nail is for topcoat layer formation.
